(19) Europäisches Patentamt
European Patent Office
Office européen des brevets

(11) Veröffentlichungsnummer: **0 172 790 B1**

(12) # EUROPÄISCHE PATENTSCHRIFT

(45) Veröffentlichungstag der Patentschrift: **29.07.92**

(51) Int. Cl.$^5$: **C07D 251/44**, C09B 62/04, //C07D251/50,C09B62/085, C09B62/09

(21) Anmeldenummer: **85810373.2**

(22) Anmeldetag: **15.08.85**

Die Akte enthält technische Angaben, die nach dem Eingang der Anmeldung eingereicht wurden und die nicht in dieser Patentschrift enthalten sind.

(54) **Verfahren zur kontinuierlichen Umsetzung von Cyanurfluorid mit Aminonaphtholsulfonsäuren.**

(30) Priorität: **21.08.84 CH 3992/84**

(43) Veröffentlichungstag der Anmeldung:
**26.02.86 Patentblatt 86/09**

(45) Bekanntmachung des Hinweises auf die Patenterteilung:
**29.07.92 Patentblatt 92/31**

(84) Benannte Vertragsstaaten:
**BE CH DE FR GB IT LI**

(56) Entgegenhaltungen:
**DE-A- 2 746 109**
**DE-A- 2 747 011**
**DE-A- 2 903 594**

(73) Patentinhaber: **CIBA-GEIGY AG**
**Klybeckstrasse 141**
**CH-4002 Basel(CH)**

(72) Erfinder: **Franke, Karlheinz, Dr.**
**Emanuel Büchel Strasse 2**
**CH-4052 Basel(CH)**
Erfinder: **Ruhlmann, Edmond, Dr.**
**Rue de Buschwiller 1**
**F-68300 Saint-Louis(FR)**

**EP 0 172 790 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung ist ein Verfahren zur kontinuierlichen Umsetzung von Cyanurfluorid mit Aminonaphtholsulfonsäuren, dadurch gekennzeichnet, dass man Cyanurfluorid mit einer wässrigen Lösung einer Aminonaphtholsulfonsäure im Molverhältnis 0,8:1 bis 1,5:1 bei einer Temperatur zwischen 0 und 50 °C gleichzeitig und kontinuierlich in einen ersten Reaktor leitet, dort eine intensive Durchmischung vornimmt, und die Reaktionsmischung anschliessend in einen zweiten Reaktor leitet, in dem nur geringe Rückvermischung aber gute radiale Vermischung eintritt, und dort die Reaktion zu Ende führt, wobei der Umsatz im ersten Reaktor maximal 50% beträgt.

Aus der DES-OS 27 46 109 ist bereits ein Verfahren zur kontinuierlichen Umsetzung von Cyanurfluorid mit Aminobenzolsulfonsäuren oder Aminonaphthalinsulfonsäuren bekannt. Gemäss den Angaben dieser DE-OS wird als Reaktor ein kontinuierlich durchflossener "Idealkessel" mit vollständiger Rückvermischung der Reaktionsmasse im Reaktor verwendet. Die dort beschriebene Arbeitsweise ist jedoch nur bedingt anwendbar für die Umsetzung von Cyanurfluorid mit Aminonaphtholsulfonsäuren, da man in diesem Falle erhebliche Mengen an Nebenprodukten erhält.

Aus der DOS 29 03 594 und der DOS 27 47 011 ist weiterhin ein Verfahren zur Umsetzung von Cyanurfluorid mit Aminonaphtholsulfonsäuren bekannt. Merkmal des dort beschriebenen diskontinuierlichen Verfahrens ist die Einhaltung eines bestimmten pH-Wertes bei der Umsetzung.

Der vorliegenden Erfindung lag die Aufgabe zugrunde, ein Verfahren zu finden, welches die kontinuierliche Umsetzung von Cyanurfluorid mit Aminonaphtholsulfonsäuren zu Monokondensationsprodukten, d.h. Verbindungen, bei denen 1 Mol Cyanurfluorid mit 1 Mol Aminonaphtholsulfonsäure reagiert, in verbesserter Ausbeute und/oder Reinheit gestattet.

Diese Aufgabe wird durch das erfindungsgemässe Verfahren gelöst, indem man die Reaktionspartner zunächst in einem ersten Reaktor miteinander vermischt und dann in einen zweiten Reaktor, in dem nur geringe Rückvermischung aber gute radiale Vermischung eintritt, die Reaktion zu Ende führt. Ueberraschenderweise erhält man bei dieser Arbeitsweise auch die Umsetzungsprodukte von 1 Mol Aminonaphtholsulfonsäure mit 1 Mol Cyanurfluorid in hoher Ausbeute und Reinheit.

Geeignete Aminonaphtholsulfonsäuren sind z. B.:
1-Amino-5-hydroxynaphthalin-7-sulfonsäure, 1-Amino-8-hydroxynaphthalin-4-sulfonsäure, 1-Amino-8-hydroxynaphthalin-3-sulfonsäure, 1-Amino-8-hydroxynaphthalin-5-sulfonsäure, 2-Amino-5-hydroxynaphthalin-7-sulfonsäure, 2-Amino-6-hydroxynaphthalin-8-sulfonsäure, 2-Amino-8-hydroxynaphthalin-6-sulfonsäure, 2-Methylamino-5-hydroxynaphthalin-7-sulfonsäure, 2-Aethylamino-5-hydroxynaphthalin-7-sulfonsäure, 2-Methylamino-8-hydroxynaphthalin-6-sulfonsäure, 2-Aethylamino-8-hydroxynaphthalin-6-sulfonsäure, 1-Amino-6-hydroxynaphthalin-3,8-disulfonsäure, 1-Amino-8-hydroxynaphthalin-3,6-disulfonsäure, 1-Amino-8-hydroxynaphthalin-2,4-disulfonsäure, 1-Amino-8-hydroxynaphthalin-4,6-disulfonsäure, 1-Amino-8-hydroxynaphthalin-3,5-disulfonsäure, 2-Amino-5-hydroxynaphthalin-7,1-disulfonsäure und 2-Amino-8-hydroxynaphthalin-3,6-disulfonsäure.

Besonders geeignet ist das erfindungsgemässe Verfahren für die Umsetzung von Cyanurfluorid mit 2-Amino-5-hydroxynaphthalin-7-sulfonsäure, 2-Amino-8-hydroxynaphthalin-6-sulfonsäure, 1-Amino-8-hydroxynaphthalin-4,6-disulfonsäure oder vor allem 1-Amino-8-hydroxynaphthalin-3,6-disulfonsäure.

Die Aminonaphtholsulfonsäuren werden als wässrige Lösungen eingesetzt. Man kann das Cyanurfluorid in grösserem Ueberschuss verwenden. Cyanurfluorid und Aminonaphtholsulfonsäure werden im Molverhältnis 0,8:1 bis 1,5:1, vorzugsweise 1:1 bis 1,2:1, insbesondere 1:1 bis 1,08:1 eingesetzt.

Vorzugsweise setzt man der Aminlösung eine Puffersubstanz zu, die bewirkt, dass während der Umsetzung, je nach Puffersubstanz, ein pH-Wert zwischen 1 und 8, vorzugsweise zwischen 1 und 4 beibehalten bleibt. Geeignete Puffersubstanzen sind z.B. Alkalimetallfluoride, insbesondere NaF. Diese Puffersubstanzen werden im allgemeinen in einer Menge von 0,5 bis 2, vorzugsweise 0,8 bis 1,2 Mol je Mol Amin eingesetzt.

Die Edukte werden gleichzeitig und kontinuierlich in einen ersten Reaktor geleitet, wo eine intensive Durchmischung erfolgt, z.B. mittels einer geeigneten Dispergiereinheit, wie einem Rotor-Stator-Mischkopf, mit ca. 1.000 bis 25.000 Umdrehungen pro Minute oder einer Ultraschallmischkammer oder einer statischen Mischvorrichtung. Die Verweilzeit in diesem ersten Reaktor soll möglichst kurz sein, sie muss jedoch so bemessen sein, dass eine genügende Durchmischung erfolgt und der Umsatz maximal 50% beträgt. Im allgemeinen beträgt die Verweilzeit im ersten Reaktor maximal 5 Sekunden, vorzugsweise maximal 1 Sekunde.

Während dieser Zeit erfolgt bereits zum Teil eine Reaktion des Cyanurfluorids mit der Aminonaphtholsulfonsäure. Es hat sich als günstig erwiesen, wenn der Umsatz in dem ersten Reaktor vorzugsweise maximal 30% beträgt. Dies wird durch geringes Volumen des ersten Reaktors bzw. durch kurze Verweilzeit

erreicht.

Aus dem ersten Reaktor wird die Reaktionsmischung in einen zweiten Reaktor geleitet und dort die Umsetzung zu Ende geführt. Als zweiter Reaktor ist generell jeder Reaktor geeignet, in dem nur geringe Rückvermischung aber gute radiale Vermischung eintritt. Vorzugsweise verwendet man einen Rohrreaktor mit gutem Pfropfströmungsprofil und arbeitet im turbulenten Strömungsbereich. Rohrreaktoren mit laminarer Strömung sind ebenfalls verwendbar, wenn sie mit geeigneten statischen Mischelementen zur Verbesserung der radialen Durchmischung ausgerüstet sind. Dieser zweite Reaktor wird gegebenenfalls gekühlt, um die Temperatur im gewünschten Bereich zu halten. Die Verweilzeit der Reaktionsmischung im zweiten Reaktor hängt u.a. von der Art der Aminonaphtholsulfonsäure und der Temperatur ab, im allgemeinen liegt sie etwa zwischen 30 Sekunden und 5 Minuten.

Die erfindungsgemässe Umsetzung kann bei Temperaturen in dem Bereich zwischen 0° und 50°C, insbesondere zwischen 0° und 20°C, ausgeführt werden.

Die erhaltenen Reaktionsprodukte aus 1 Mol Aminonaphtholsulfonsäure und 1 Mol Cyanurfluorid können isoliert werden, sie werden jedoch vorzugsweise ohne Zwischenisolierung weiterverarbeitet, z.B. zu Reaktivfarbstoffen durch Umsetzung mit einem aminogruppenhaltigen Farbstoff oder durch Umsetzung mit einem aromatischen Amin und nachfolgende Kupplung mit einer Diazoniumverbindung. Diese Weiterverarbeitung kann diskontinuierlich oder kontinuierlich auf bekannte Art und Weise durchgeführt werden.

Nach dem erfindungsgemässen Verfahren erhält man die Kondensationsprodukte aus Cyanurfluorid und Aminonaphtholsulfonsäuren in vielen Fällen in deutlich höherer Reinheit als nach den bisher üblichen Verfahren. Dies wirkt sich positiv auf die Qualität der aus den Konsensationsprodukten hergestellten Reaktivfarbstoffe aus, da üblicherweise die Kondensationsprodukte ohne Zwischenreinigung weiterverarbeitet werden.

Die folgenden Beispiele dienen der Erläuterung der Erfindung. Die Temperaturen sind in Grad Celsius angegeben.

<u>Beispiel 1:</u>

In einen ersten Reaktor (Dispergiereinheit) werden gleichzeitig und koninuierlich über getrennte Zuleitungen 4,7 ml/min Cyanurfluorid mit einer Temperatur von ca. 25°C sowie 300 ml/min einer wässrigen Lösung von 0°C, enthaltend 15,95 g 1-Amino-8-hydroxynaphthalin-3,6-disulfonsäure, 2,1 g NaF und 5 ml NaOH 30%, eingespeist.

In dem Reaktor, der ein Volumen von ca. 1 ml aufweiset, wird durch eine Dispergiervorrichtung während einer Verweilzeit von 0,2-1 sec bei hoher Turbulenz eine gleichmässige, schnelle Suspendierung der dispersen Phase erreicht. Gleichzeitig findet hier ca. 20-50% Umsatz statt.

Aus dem ersten Reaktor wird die Reaktionsmischung durch einen gekühlten Rohrreaktor mit guten Pfropfströmungsprofil, dessen Volumen ca. 340 ml beträgt, geleitet. Die Verweilzeit in dem Rohr-Reaktor beträgt etwa 60 sec. Das Reaktionsgemisch das den Reaktor mit einer Temperatur von 3° verlässt, enthält die Verbindung der Formel

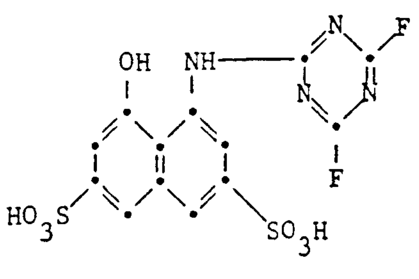

in einer Ausbeute von 85%, bezogen auf eingesetzte 1-Amino-8-hydroxynaphthalin-3,6-disulfonsäure.

Die erhaltene Reaktionsmischung wird in einen Rührkessel geleitet und dort nach der folgenden Vorschrift in einen roten Reaktivfarbstoff überführt:

Zu der Reaktionsmischung gibt man pro Mol des vorstehend beschriebenen Kondensationsproduktes eine Lösung, enthaltend 1 Mol p-Chloranilin, 500 ml Wasser und 100 ml Salzsäure 32% und stellt innerhalb von 10-20 Minuten mit 20%-iger NaOH einen pH-Wert von 6.0 ein.

Die erhaltene Lösung gibt man zu einer Diazoniumsalz-Suspension von 0-5°, welche durch Diazotierung auf übliche Weise von 1 Mol 2-Naphthylamin-1,5-disulfonsäure erhalten wurde. Bei max. 10° stellt

man unter intensivem Rühren mit 30%-iger Natronlauge einen pH-Wert von 7,5 ein und rührt noch 1 Stunde bei pH 7,5 und 10°.

Man erhält den Farbstoff der Formel

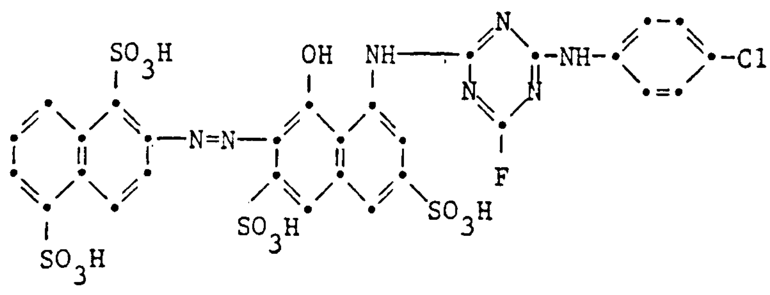

mit einer Ausbeute von 80-85%, bezogen auf eingesetzte 1-Amino-8-hydroxynaphthalin-3,6-disulfonsäure. Der Farbstoff färbt Cellulosematerial in sehr nassechtem roten Farbton.

Nach den bisher üblichen Verfahren, d.h. bei Durchführung der Kondensation von Cyanurfluorid mit 1-Amino-8-hydroxynaphthalin-3,6-disulfonsäure im Rührkessel erhält man bei im übrigen gleicher Arbeitsweise den vorstehend beschriebenen Farbstoff nur in etwa 60-65% Ausbeute und zudem mit roten Verbindungen verunreinigt, welche u.a. schlechte Nassechtheiten der auf Cellulosematerial erzielbaren Färbungen zur Folge haben.

Verfährt man wie in Beispiel 1 angegeben, wobei man dafür sorgt, dass das Reaktionsgemisch den Reaktor mit einer Temperatur von 20°C verlässt, und verarbeitet sofort weiter, erhält man das Endprodukt der oben angegebenen Formel in gleicher Ausbeute.

Im Reaktionsansatz kann statt NaOH auch LiOH eingesetzt und dafür das NaF fortgelassen werden.

Beispiel 2

Wird in den ersten Reaktor gleichzeitig und kontinuierlich über getrennte Zuleitungen 4,9 ml/min Cyanurfluorid sowie 300 ml/min einer wässrigen Lösung von 0° enthaltend 11,95 g 2-Amino-5-hydroxynaphthalin-7-sulfonsäure und 25 ml 2N Lithiumhydroxid eingespeist und verfährt unter den gleichen Bedingungen wie in Beispiel 1, so erhält man in guter Ausbeute die Verbindung der Formel

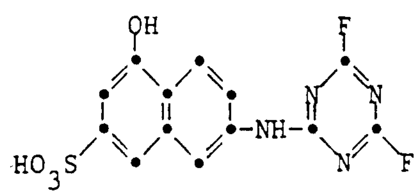

Die erhaltene Reaktionsmischung wird in einen Rührkessel geleitet und dort nach der folgenden Vorschrift in einen orangen Reaktivfarbstoff überführt:

Pro Mol des vorstehend beschriebenen Kondensationsproduktes gibt man 1 bis 1,2 Mol N-Aethylanilin zu und stellt innerhalb von 10 bis 30 Minuten mit 20%iger NaOH einen pH-Wert von 6,0 ein. Die erhaltene Lösung gibt man zu einer Diazoniumsalz-Suspension von 0 bis 5°, welche durch Diazotierung auf übliche Weise von 1 Mol 2-Naphthylamin-1,5-disulfonsäure erhalten wurde. Bei max. 10° stellt man mit 30%iger NaOH einen pH-Wert von 7,5 ein. Nach Aussalzen und Abfiltrieren erhält man in guter Qualität und Ausbeute den orangen Reaktivfarbstoff der Formel

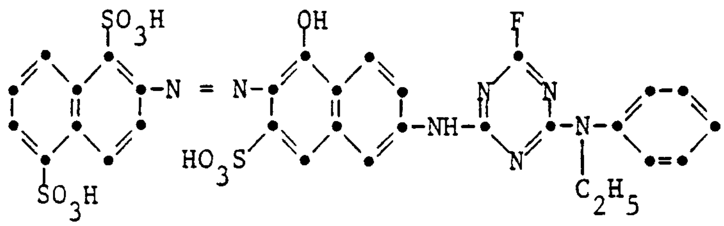

.

## Patentansprüche

1. Verfahren zur Umsetzung von Cyanurfluorid mit einer Aminonaphtholsulfonsäure , dadurch gekennzeichnet, dass man Cyanurfluorid und eine wässrige Lösung einer Aminonaphtholsulfonsäure im Molverhältnis 0,8:1 bis 1,5:1 bei einer Temperatur zwischen 0 und 50 ° C gleichzeitig und kontinuierlich in einen ersten Reaktor leitet, dort eine intensive Durchmischung vornimmt, und die Reaktionsmischung anschliessend in einen zweiten Reaktor leitet, in dem nur geringe Rückvermischung aber gute radiale Vermischung eintritt, und dort die Umsetzung zu Ende führt, wobei der Umsatz in dem ersten Reaktor maximal 50% beträgt.

2. Verfahren gemäss Anspruch 1, dadurch gekennzeichnet, dass man die Umsetzung bei 0 bis 20 ° C ausführt.

3. Verfahren gemäss Anspruch 1, dadurch gekennzeichnet, dass die intensive Durchmischung innerhalb von maximal 5 Sekunden, vorzugsweise maximal 1 Sekunde erfolgt.

4. Verfahren gemäss Anspruch 1, dadurch gekennzeichnet, dass in dem ersten Reaktor der Umsatz maximal 30% beträgt.

5. Verfahren gemäss Anspruch 1, dadurch gekennzeichnet, dass als zweiter Reaktor ein Rohr-Reaktor mit gutem Pfropfströmungsprofil verwendet wird.

6. Verfahren gemäss Anspruch 1, dadurch gekennzeichnet, dass Cyanurfluorid und Aminonaphtholsulfonsäure im Molverhältnis 1:1 bis 1,2:1, insbesondere 1:1 bis 1,08:1, eingesetzt werden.

7. Verfahren gemäss Anspruch 1, dadurch gekennzeichnet, dass als Aminonaphtholsulfonsäure 2-Amino-5-hydroxynaphthalin-7-sulfonsäure, 2-Amino-8-hydroxynaphthalin-6-sulfonsäure, 1-Amino-8-hydroxynaphthalin-4,6-disulfonsäure oder insbesondere 1-Amino-8-hydroxynaphthalin-3,6-disulfonsäure verwendet wird.

8. Verfahren gemäss Anspruch 1, dadurch gekennzeichnet, dass die Umsetzung in Gegenwart eines Puffers bei einem pH-Wert zwischen 1 und 8, insbesondere bei einem pH-Wert zwischen 1 und 4 durchgeführt wird.

9. Verfahren gemäss Anspruch 8, dadurch gekennzeichnet, dass als Puffer ein Alkalimetallfluorid, vorzugsweise NaF verwendet wird.

10. Verfahren gemäss einem der Ansprüche 8 oder 9, dadurch gekennzeichnet, dass man den Puffer in einer Menge von 0,5 bis 2 Mol, vorzugsweise 0,8 bis 1,2 Mol, je Mol Aminonaphtholsulfonsäure einsetzt.

## Claims

1. A process for the reaction of cyanuric fluoride with an aminonaphtholsulfonic acid, which comprises passing cyanuric fluoride and an aqueous solution of an aminonaphtholsulfonic acid, in a molar ratio of 0.8:1 to 1.5:1 and at a temperature between 0 and 50 ° C, simultaneously and continuously into a first reactor, performing thorough mixing there, then passing the reaction mixture into a second reactor in which there is only little backmixing but good radial mixing, and completing the reaction there, the

conversion in the first reactor being at most 50%.

**2.** A process according to claim 1, wherein the reaction is carried out at 0 to 20 °C.

**3.** A process according to claim 1, wherein the thorough mixing is effected within at most 5 seconds, preferably at most 1 second.

**4.** A process according to claim 1, wherein the conversion in the first reactor is at most 30%.

**5.** A process according to claim 1, wherein the second reactor is a tubular reactor having a good plug flow profile.

**6.** A process according to claim 1, wherein cyanuric fluoride and aminonaphtholsulfonic acid are used in a molar ratio of 1:1 to 1.2:1, in particular 1:1 to 1.08:1.

**7.** A process according to claim 2, wherein the aminonaphtholsulfonic acid used is 2-amino-5-hydroxynapthalene-7-sulfonic acid, 2-amino-8-hydroxynaphthalene-6-sulfonic acid, 1-amino-8-hydroxynaphthalene-4,6-disulfonic acid or in particular 1-amino-8-hydroxynaphthalene-3,6-disulfonic acid.

**8.** A process according to claim 1, wherein the reaction is carried out in the presence of a buffer at a pH between 1 and 8, in particular at a pH between 1 and 4.

**9.** A process according to claim 8, wherein the buffer used is an alkali metal fluoride, preferably NaF.

**10.** A process according to one of claims 8 or 9, wherein the buffer is used in an amount of 0.5 to 2 mol, preferably 0.8 to 1.2 mol, per mol of aminonaphtholsulfonic acid.

**Revendications**

**1.** Procédé pour la conversion du fluorure de cyanuryle avec un acide aminonaphtolsulfonique, caractérisé en ce qu'on introduit, simultanément et en continu, du fluorure de cyanuryle et une solution aqueuse d'un acide aminonaphtolsulfonique, selon un rapport molaire de 0,8:1 à 1,5:1, à une température comprise entre 0 et 50 °C, dans un premier réacteur où on effectue un mélange intime, et ensuite, on amène le mélange réactionnel dans un deuxième réacteur dans lequel se produit un remélange seulement réduit, mais un bon mélange radial, et où la conversion s'effectue jusqu'à la fin, le taux de conversion dans le premier réacteur étant d'au plus 50 %.

**2.** Procédé selon la revendication 1, caractérisé en ce qu'on effectue la conversion à une température comprise entre 0 et 20 °C.

**3.** Procédé selon la revendication 1, caractérisé en ce que le mélange intime est effectué en un temps maximal de 5 secondes, de préférence en un temps maximal de 1 seconde.

**4.** Procédé selon la revendication 1, caractérisé en ce que le taux de conversion dans le premier réacteur est d'au plus 30 %.

**5.** Procédé selon la revendication 1, caractérisé en ce qu'on utilise comme deuxième réacteur, un réacteur tubulaire ayant un bon profil d'écoulement piston.

**6.** Procédé selon la revendication 1, caractérisé en ce qu'on utilise le fluorure de cyanuryle et l'acide aminonaphtolsulfonique selon un rapport molaire de 1:1 à 1,2:1, en particulier de 1:1 à 1,08:1.

**7.** Procédé selon la revendication 1, caractérisé en ce qu'on utilise comme acide aminonaphtolsulfonique, l'acide 2-amino-5-hydroxynaphtalène-7-sulfonique, l'acide 2-amino-8-hydroxynaphtalène-6-sulfonique, l'acide 1-amino-8-hydroxynaphtalène-4,6-disulfonique ou, plus particulièrement, l'acide 1-amino-8-hydroxynaphtalène-3,6-disulfonique.

**8.** Procédé selon la revendication 1, caractérisé en ce que la conversion est réalisée en présence d'un

tampon, à un pH compris entre 1 et 8, en particulier entre 1 et 4.

**9.** Procédé selon la revendication 8, caractérisé en ce qu'on utilise comme tampon, un fluorure de métal alcalin, de préférence NaF.

**10.** Procédé selon la revendication 8 ou 9, caractérisé en ce qu'on utilise le tampon à raison de 0,5 à 2 moles, de préférence de 0,8 à 1,2 mole, par mole d'acide aminonaphtolsulfonique.